# EUROPEAN PATENT SPECIFICATION

(11) **EP 1 998 784 B1**
(45) Date of publication and mention of the grant of the patent: **22.07.2009**
(21) Application number: 07848589.3
(22) Date of filing: 18.12.2007
(51) Int. Cl.: A61K 31/565, A61K 47/10, A61K 47/24, A61K 9/00, A61P 17/02

(54) **MEDICAMENTS AND METHODS COMPRISING A COMPOUND THAT PROMOTES OESTROGENIC ACTIVITY FOR WOUND HEALING**
MEDIKAMENTE UND VERFAHREN MIT EINER VERBINDUNG ZUR FÖRDERUNG VON ÖSTROGEN-AKTIVITÄTEN ZUR WUNDHEILUNG
MÉDICAMENTS ET PROCÉDÉS FAISANT APPEL À UN COMPOSÉ QUI STIMULE L'ACTIVITÉ OESTROGÉNIQUE POUR LA CICATRISATION DES PLAIES

(30) Priority: 23.12.2006 GB 0625962; 16.03.2007 US 723192
(43) Date of publication of application: 10.12.2008
(73) Proprietor: Renovo Limited, Manchester M13 9XX (GB)
(72) Inventor: FERGUSON, Mark William James, Manchester M13 9XX (GB); OCCLESTON, Nicholas, Manchester M13 9XX (GB); O'KANE, Sharon, Manchester M13 9XX (GB); HADFIELD, Peter, Manchester M13 9XX (GB)
(74) Representative: Armstrong, Iain Cheshire
(86) International application number: PCT/GB2007/004852
(87) International publication number: WO 2008/078071

(56) References cited:
- WO-A-98/03180
- US-A1- 2004 132 701
- MAHMOUD A ET AL: "Cutaneous estradiol permeation, penetration and metabolism in pig and man" SKIN PHARMACOLOGY AND PHYSIOLOGY, vol. 18, no. 1, 2005, pages 27-35, XP009096381 ISSN: 1660-5527(print) 1660-5535(ele
- HALL ET AL: "Estrogen and skin: The effects of estrogen, menopause, and hormone replacement therapy on the skin" JOURNAL OF THE AMERICAN ACADEMY OF DERMATOLOGY, C.V. MOSBY, ST. LOUIS, MO, US, vol. 53, no. 4, October 2005 (2005-10), pages 555-568, XP005120118 ISSN: 0190-9622

## Description

The present invention relates to the manufacture of medicaments. In particular, the invention relates to the manufacture of medicaments for accelerating the healing of wounds. In particular the invention relates to medicaments for the acceleration of healing of skin wounds.

The skin is the most frequently injured of the body's organs. By virtue of its location, the skin is in constant contact with the external environment, and as a result is the organ most frequently exposed to environmental, and other, damage.

Wounds, such as skin wounds, may arise as a result of many different forms of damage. Such damage may impair or entirely destroy the function of the injured organ or tissue, and the outcome of such damage depends on the nature and role of the tissue or organ affected.

The wound healing response is most commonly described with reference to the healing of - skin wounds. The response involves a sequence of overlapping reparative processes. One of the most important of these processes in terms of influencing the rate of wound healing is that of re-epithelialisation. This process is responsible for the re-constitution of a functional epithelial barrier (such as the epidermis) at the site of a wound.

Skin wounds primarily re-epithelialise "from the outside in", that is to say that keratinocytes (epithelial cells) from the unwounded skin surrounding the damaged area proliferate and migrate to cover the tissue at the wound site. This migration of keratinocytes means that the area around the edge of the wound is the first to heal, and the progress of the keratinocytes over the damaged area provides a useful index by which the progress of wound healing may be measured. In wounds, such as partial thickness wounds, where epidermal appendages such as hair follicles remain, these may provide an additional source of epithelial cells contributing to the re-epithelialisation process.

Wounding of organs such as the skin gives rise to a number of undesirable effects. One noticeable effect is that the presence of a wound impairs the barrier function of the skin. This increases the rate at which fluids are lost from the wounded area, which can be a particular concern in the case of wounds covering large areas. It is common for burns victims to suffer severe, potentially life-threatening, dehydration as a result of fluid loss through the damaged skin. The loss of the skin's barrier function also increases the risk of ingress and infection by pathogens such as bacteria and fungi.

Wounds are painful, even aside from the events associated with their formation, and delays in the healing of wounds may be associated with extended incidences of pain to the suffered. Wounds can also decrease the mechanical function of the injured area.

In the light of the above, it will be seen that the acceleration of healing of wounds is advantageous for many different reasons. However, despite the desirability of accelerating the healing of wounds, there remains a well recognised requirement for new, alternative, and more effective, medicaments and methods by which such acceleration may be attained.

There is significant variation in the range of therapies currently used in attempts to accelerate the healing of wounds. At their most basic level these may merely be concerned with preventing or stopping blood loss as a result of the wound, and preventing infection of the wound in an attempt to promote healing.

More advanced therapies known from the prior art include the use of wound management techniques, accelerants for wound closure and skin substitutes for graft augmentation. Debridement is often used for the treatment of chronic wounds to remove non-viable tissue, this usually takes the form of mechanical/surgical or enzymatic removal of the effected tissue. Surgical excision rapidly clears the area of the affected tissue but can be extremely painful, it is also non-specific and can therefore lead to complications induced by the increased injury to the area. Addition of enzymatic agents such as collagenase, papain-urea and bromelain to clear the wound area have yielded mixed efficacy in current studies.

Currently management of the rate of wound closure is primarily reliant upon the initial dressing of the wound, and wound management via the addition of antimicrobial agents to the wound site. A number of dressings are used to clear the site of extraneous fluid/tissue either by absorption or using vacuum-assisted closure. The disadvantage of dressings of this nature is the need to retain the dressing in position, which may be difficult depending on wound site. Adding silver as an antimicrobial agent to the dressing has been extensively used, with a number of products on the market, but these products have exhibited variable efficacy in practice. Skin substitutes offer a method for temporary wound coverage but ultimately auto grafting is required due to the survival of the cultured sheets of cells.

A number of targets exist for the acceleration of wound healing, these include growth factors, oxidised regenerated cellulose/collagen matrices, adenosine A_{2A} agonists and recombinant lactoferrin. These act in a variety of ways; the collagen matrix acts to inhibit the negative impact of proteases and oxygen free radicals whilst promoting the activity of growth factors within the wound area; the lactoferrin acts to promote IL-18 up regulation within the wound. Gene therapy is now being utilised for the treatment of impaired wound healing, with the modulation of growth factors being the primary target. This is an extremely underdeveloped field and requires further research to investigate the efficacy of this approach.

The management of wounds such as split thickness skin graft donor sites (reviewed in Rakel *et al.* 1998) may merely involve leaving the graft donor site exposed and untreated, or may alternatively make use of treatments such as the application of dressings (typically gauze dressings, which may be used alone or impregnated with a variety of anti-infective agents, alginates, hydrocolloids, synthetic composite membranes, transparent films or honey), application of artificial skin (which may be generated from the individuals own epidermis), application of allografts (typically bovine or porcine allografts) or application of ointments (typically ointments containing silver based compounds as anti-infective agents).

The absence of a single universally accepted method for accelerating the healing of wounds is indicative of the need for novel medicaments by which such acceleration may be effected. It is well recognised that there are failings and disadvantages associated with many of the current therapies available. Even in the case of relatively successful therapies, there is scope for improvement in terms of increased efficacy, or other parameters.

There are a number of adverse effects associated with current regimes used in the management of wounds. These include protracted healing times, which may ultimately lead to the development of chronic wounds. Other undesirable effects relate to the qualities of the replacement tissues or organs that are generated via the healing process. These may frequently be rougher and/or thinner than those originally present. Slow rates of healing may be associated with increased rates of infection. These may be increased in the case of treatments using allograft materials, which may harbour agents of infection, such as bacteria, fungi, prions or viruses. Delays in wound healing may also be associated with increased edema, erythema and pain.

One effective therapy for the promotion of re-epithelialisation of skin wounds comprises the administration to the wound of a compound that promotes oestrogenic activity. US 2004/132701 A1. WO 98/03180 A and Hall et al. ("Estrogen and skein: The effects of estrogen, menopause, and hormone replacement therapy on the skin" - Journal of The American Academy of Dermatology: vol. 53, no. 4, October 2005, pages 555-568) all disclose that compounds that promote oestronenic activity may be used for accelerating the healing of wounds.

It is an object of certain aspects of the invention to provide new medicaments that may be used to accelerate the healing of wounds. It is an object of certain aspects of the invention to provide alternative medicaments that may be used to accelerate the healing of wounds. It is an object of certain aspects of the invention to provide medicaments that may be used to accelerate the healing of wounds with greater efficiency than is achieved by the prior art. It is an object of certain aspects of the invention to provide medicaments that may be used to accelerate the healing of wounds to a grater extent than may be achieved by the prior art. It is an object of certain aspects of the invention to provide more cost effective medicaments for the acceleration of wound healing.

In a first aspect, the invention provides the use of an oestrogen in the manufacture of a medicament, wherein the medicament comprises said oestrogen provided in an injectable carrier solution, the injectable carrier solution comprising an alcohol and a phosphate buffer. This aspect of the invention also provides an oestrogen for use as a medicament, wherein the medicament comprises said oestrogen provided in an injectable carrier solution, the injectable carrier solution comprising an alcohol and a phosphate buffer. Medicaments in accordance with the first aspect of the invention may be used for the acceleration of wound healing.

In a second aspect, the invention provides the use of an oestrogen in the manufacture of a medicament for accelerating the healing of wounds, wherein the medicament comprises said oestrogen provided in a solution comprising an alcohol and a phosphate buffer. This second aspect of the invention also provides an oestrogen for use as a medicament for accelerating the healing of wounds, wherein the medicament comprises said oestrogen provided in a solution comprising an alcohol and a phosphate buffer. Medicaments in accordance with the second aspect of the invention may be used for the acceleration of wound healing.

It is preferred that the wounds, the healing of which is to be accelerated by medicaments of the invention, may be skin wounds.

The present invention is based on the very surprising finding that the provision of oestrogens in a solution comprising an alcohol and a phosphate buffer has a markedly beneficial effect on the rate of wound healing. This effect is more pronounced than the increase in the rate of wound healing that may be achieved using compositions described in the prior art that comprise a compound that promotes oestrogenic activity, but not in the presence of an alcohol and a phosphate buffer. This increased effectiveness confers notable advantages over the prior art (such as US 2004/132701 A1, WO 98/03180 A) in terms of the acceleration of healing that can be brought about using medicaments of the invention.

Mahmoud et al. ("Cutaneous estradiol permeation, penetration and metabolism in pig and man" - Skin Pharmacology and Physiology, vol. 18, no. 1, 2006, pages 27-35) had previously suggested that ethanol seems to enhance the permeation of oestradiol across the human skin, but did not disclose that a composition comprising ethanol would improve wound healing.

It is preferred that the medicaments of the invention comprise injectable medicaments, In particular it may be preferred that the injectable medicament be suitable for intradermal injection.

The oestrogen may preferably be 17β-oestradiol. However, the inventors believe that other oestrogens may be used in the medicaments of the invention, and a suitable oestrogen may be selected from the group consisting of: ethinylyoestradiol, dienoestrol, mestranol, oestradiol, 17β-oestradiol, oestriol, conjugated oestrogens, and phytoestrogens.

Medicaments of the invention comprise a phosphate buffer. The total concentration of phosphate present in a medicament of the invention may, for example, be between 1 mM and 50 mM. The total concentration of phosphate present in a medicament of the invention may preferably be approximately 4.2 mM.

Potassium dihydrogen phosphate may be used as part of a suitable phosphate buffer in a medicament of the invention. A medicament of the invention may comprise between 0.1 mM and 20 mM potassium dihydrogen phosphate. Preferably, a medicament of the invention may comprise potassium dihydrogen phosphate at a concentration of between 1.20 mM and 1.54 mM, most preferably at a concentration of approximately 1.5 mM.

Disodium phosphate may also be used as part of a suitable phosphate buffer in a medicament of the invention. The concentration of disodium phosphate may preferably be between 0.1 mM and 20 mM, more preferably between 2.70 mM and 3.06 mM, and is most preferably approximately 2.7 mM.

The concentration of the alcohol in a medicament of the invention is preferably between 0.17 M and 1.71 M. Preferably the concentration of alcohol in a medicament of the invention may be between 0.42 M and 1.275 M. The concentration of the alcohol in a medicament of the invention is most preferably approximately 0.85M.

Various different alcohols may be used in the medicaments of the invention. It is preferred that the alcohol is selected from the group consisting of ethanol or higher water-miscible alcohols such as isopropyl alcohol or benzyl alcohol or other suitable alcohols, many of which are known to those skilled in the art as solubilisers/excipients.

A medicament of the invention will preferably comprise a source of sodium ions. A suitable source of sodium ions may provide up to 0.9% (w/v) of the medicament Sodium chloride may constitute a suitable source of such sodium ions. Alternatively, or additionally, it may be preferred that the medicament comprises a source of potassium ions. Potassium chloride may constitute a suitable source of potassium ions.

In the case that a medicament of the invention comprises sodium chloride, it may be preferred that the concentration of sodium chloride is between 130 mM and 180 mM. It may be particularly preferred that the concentration of sodium chloride is approximately 154 mM.

In the case that a medicament of the invention comprises potassium chloride, it may be preferred that the concentration of potassium chloride is between 130mM and 180mM. It may be particularly preferred that the concentration of potassium chloride is approximately 160mM.

A medicament of the invention may preferably have a pH of between 6.7 and 7.7. It is particularly preferred that the pH of a medicament of the invention is approximately 7.2.

The acceleration of healing of wounds within the context of the present invention may be understood to encompass any increase in the rate of healing of a treated wound as compared to the rate of healing occurring in a control-treated or untreated wound. The rate of healing wounds attained in accordance with the invention may readily be compared with that taking place in control-treated or untreated wounds using any suitable model of wound healing known in the art. Suitable models in which the rate of wound healing may be assessed are set out elsewhere in the specification.

Accelerated healing of a wound achieved using the medicaments of the invention may preferably lead to a treated wound healing at a rate at least 5% faster than an untreated wound, preferably at a rate at least 10% faster, more preferably at least 15%, 20% or 25% faster; yet more preferably at least 50% faster, still more preferably at least 75% faster, and most preferably 100% (or more) faster. Suitable methods by which acceleration of the healing of wounds may be quantified to assess improvements in the rate of healing are described elsewhere in the specification.

In relation to the second aspect of the present invention a "therapeutically effective amount of oestrogen" is an amount of such a compound that is sufficient to accelerate healing of a wound of a subject to whom the amount is administered. Suitable therapeutically effective amounts that may be utilised in medicaments of the invention are considered elsewhere in the specification. Purely by way of example, a therapeutically effective amount of an oestrogen such as 17β-oestradiol, may comprise between 0.005µg and 4µg per wound centimetre, preferably between 0.1 µg and 1 µg per wound centimetre, and particularly between 0.1µg and 0.2µg per wound centimetre.

It may be preferred that a medicament in accordance with any aspect of the present invention be one in which the medicament provides an amount of oestrogenic activity equivalent to that produced by a tissue concentration of between 1µM and 10µM of 17β-oestradiol, preferably between 3µM and 7µM and most preferably between 3.3µM and 6.6µM. Based upon the anticipated spread of drug using a dye experiment the 0.1µg/100µL dose described elsewhere in the specification gives rise to a tissue concentration of 3.3µM and the 0.2µg/100µL dose gives rise to a tissue concentration of 6.6µM.

Various terms that are used in the present disclosure to describe the invention will now be explained further. The definitions provided below may be expanded on elsewhere in the specification as appropriate, and as the context requires.

### "Medicaments of the invention"

For the purposes of the present disclosure, medicaments of the invention should be taken as encompassing any medicament manufactured in accordance with any aspect or embodiment of the invention. Suitable compositions, formulations and routes of delivery that may be used for medicaments of the invention are considered in more detail at various points in the specification. All medicaments of the invention will comprise an oestrogen provided in a solution comprising an alcohol and a phosphate buffer. Generally it will be preferred that such solutions are aqueous solutions (i.e. solutions in which the major diluent is water).

### "Active compound"

Except for where the context requires otherwise, for the purposes of the present disclosure an "active compound" should be taken to be any oestrogen that accelerates healing of wounds, in accordance with the present disclosure. Examples of suitable active compounds are provided elsewhere in the present disclosure, and favoured active compounds include 17β-oestradiol.

### "Therapeutically effective amount"

The term "therapeutically effective amount" as used in the context of the present disclosure when referring to a medicament of the invention, or an amount of an active compound (in accordance with the definition offered elsewhere) refers to an amount of the medicament, or of an active compound, sufficient to accelerate the healing of a wound. A therapeutically effective amount in the context of the invention may preferably be an amount sufficient to accelerate healing to a quantifiable extent considered elsewhere in the specification.

Generally it may be preferred that a medicament of the invention provides a therapeutically effective amount of between 0.02 µg and 0.3 µg of 17β-oestradiol per centimetre of wound. Preferred therapeutically effective amounts of oestrogens (such as 17β-oestradiol) suitable for use in the medicaments of the invention are considered in greater detail elsewhere in the specification. A preferred therapeutically effective amount will be an amount capable of increasing the rate of re-epithelialisation of a wound, and/or capable of increasing the rate of contraction of a wound.

### "Tissue concentration"

For the purposes of the present disclosure, the tissue concentration of an oestrogen should be taken to be the peak concentration of the compound achieved after administration of the compound to a wound site, or to a site where wounding will occur. Preferably tissue concentration may be defined with reference to a concentration of 17β-oestradiol in skin.

Tissue concentration may be determined by any appropriate method known to those skilled in the art. For example, the tissue concentration of 17β-oestradiol may be determined by use of assays such as ELISA (enzyme linked immunosorbent assay) to determine the amount of 17β-oestradiol present in a known volume of a tissue to which the compound has been administered. A suitable ELISA may, for instance, be conducted using an extract comprising the 17β-oestradiol from within the known volume of tissue, this method may be manipulated to include alternative strategies for the analysis of 17β-oestradiol using RIA (radioimmunoassay), HPLC or mass spectrometry. The method of RIA for 17β-oestradiol analysis is well characterised for the analysis of 17β-oestradiol from various body sites. A number of commercial radioimmunoassay kits are available for the analysis of 17β-oestradiol in human samples (Stanczyk et al, 68 (2003) 1173-1178). Extraction and derivitisation of the 17β-oestradiol from tissue/fluid samples is the most suitable method for LC-MS analysis, as described by Nelson et al (Clinical Chemistry 50:2 (2004) 373-384). A comparison of radioimmunoassay and mass spectrometry for 17β-oestradiol analysis has been carried out by Dorgan et al (Steroids 67 (2002) 151-158). Further work on GC-MS analysis has been carried out as described by Lee et al, (J Clin Endocrinol Metab 91:10 (2006) 3791-7).

The tissue concentration of an oestrogen may be taken to encompass the concentration of the compound resulting both through administration of the active compound, and, if applicable, the concentration occurring as a result of the presence of the naturally occurring (endogenous) compound.

### "Promotion of oestrogenic activity"

In the context of the present disclosure "promotion of oestrogenic activity" may be considered to encompass any promotion or increase in oestrogenic activity that is achieved on administration of an active compound. The oestrogenic activity to be promoted may preferably be the acceleration of healing of a wound, however other activities may also be investigated in the assessment of oestrogenic activity. It will immediately be appreciated that, once oestrogenic activity has been assessed, it is then a simple matter to determine whether or not such activity is, or has been, promoted on administration of a compound that may putatively have oestrogenic activity.

Oestrogenic activity may be assessed with reference to any one of a number of assays well known to the skilled person. Suitable assays include both *in vivo* and *in vitro* assays. For example, oestrogenic activity may be assessed *in vivo* by means of a rodent uterotrophic assay. Briefly, oestrogenic activity is demonstrated in such an assay by the ability of a compound to increase the weight of the uteruses of experimental rodents (such as rats). Compounds to be investigated for the ability to promote oestrogenic activity may be administered by dermal or oral routes.

Suitable *in vitro* assays for the assessment of oestrogenic activity may include the MCF-7 human breast cancer cell assay. In this assay oestrogenic activity of a compound is demonstrated by the ability of the compound to induce increased proliferation of the breast cancer cells.

In the case of either *in vivo* or *in vitro* assays, compounds having known oestrogenic activity, such as 17β-oestradiol, may be used as positive controls, and to produce dose response curves by which oestrogenic activity may be quantified. Such quantification may be particularly useful in assessing whether or not an oestrogen has activity making it suitable for use in accordance with the present invention.

### "Topical medicament"

A "topical medicament", for the purposes of the present disclosure, is to be construed as a medicament that is applied at a site where it is intended to have its effect. Preferred topical medicaments suitable for use in accordance with the present invention include, but are not necessarily limited to, injectable solutions administered by local injections (e.g. intradermal injections). Other preferred topical medicaments may be suitable of administration to the surface of an area to be treated, for example in the forms of liquid sprays, irrigation solutions or creams.

Topical medicaments (i.e. those having their effect at the site, and preferably in the tissue, to which they are administered) will generally be preferred over medicaments or routes of administration associated with systemic administration of agents. For example, injectable medicaments of the invention may be for use in localised routes of administration, such as intradermal injection, rather than systemic routes of administration (such as intravenous, intraperitoneal, or subcutaneous injection).

### "Wounds"

For the purpose of the present disclosure wounds will primarily be described with reference to skin wounds, which comprise preferred wounds, the healing of which may be accelerated in accordance with the present invention. However, the skilled person will appreciate that the acceleration of wound healing in accordance with the invention should preferably not be limited to skin wounds. The inventors believe that healing of wounds may be accelerated in wounds of all tissues, though these will preferably be of tissues other than those of the urinogenitary organs (here considered to comprise in particular the organs of the reproductive tract and the bladder).

Skin wounds, the healing of which may be accelerated using the medicaments of the invention, include both chronic wounds and acute wounds. Examples of suitable chronic or acute wounds the healing of which may be accelerated in accordance with the invention are set out elsewhere in the specification.

The medicaments of the invention may be used to accelerate the healing of full thickness or partial thickness wounds (respectively wounds in which the epithelial layer is either totally or partly compromised). Preferred examples of partial thickness wounds the healing of which may be accelerated using the medicaments of the invention include so called "skin peels" or dermabrasion (in which the epidermal layer is totally or partially removed by chemical or mechanical means) and split thickness skin grafts.

Examples of specific wounds, other than those of the skin, which may benefit from accelerated healing of wounds in accordance with the present invention include, but are not limited to, those selected from the group consisting of: wounds of the eye (including wounds of the cornea, and acceleration of healing of wounds resulting from eye surgery such as LASIK or PRK surgery); wounds of blood vessels; wounds of the peripheral or central nervous system (where increasing the rate of healing of wounds may enhance the capability for neuronal reconnection); wounds of tendons, ligaments or muscle; wounds of the oral cavity, including the lips and palate; wounds of the internal organs such as the liver, heart, brain and digestive tissues; and wounds in body cavities such as the abdominal cavity, pelvic cavity and thoracic cavity.

It is particularly preferred that the medicaments of the invention be used to accelerate the healing of skin wounds.

### "Accelerating the healing of wounds"

"Acceleration of the healing of wounds", or "acceleration of wound healing" in the context of the present disclosure should be taken to encompass any increase in the rate at which a wound is covered, and so healed. An acceleration of wound healing may be most readily demonstrated by an increased rate of re-epithelialisation of a wound. Such an increase in the rate at which the epithelial covering (for example the epidermis) is repaired or regenerated will indicate that the healing of the wound in question has been accelerated.

Acceleration of the healing of wounds should preferably be distinguished from closure of a wound brought about by wound contraction. In this case contraction of elements (generally thought to be myofibroblasts) located within and around the periphery of the wound leads to a reduction in the surface area of the wound, but this may not be associated with covering of the wound by epithelium. Failure of the wound to re-epithelialise, even though the surface area of the wound may be reduced, will generally mean that tissue function remains impaired, and so the wound will, for the present purposes, not be regarded as having healed.

Accelerated healing of wounds in the context of the present disclosure may also be distinguished from "filling" of wounds that may occur through the generation of granulation tissue. Although granulation tissue may serve to fill a wound cavity, or the base of an open or chronic wound, the wounded area will still generally lack a functional epithelial layer, and so will not be regarded as healed since its function will remain impaired.

Accelerated healing of a wound in accordance with the present disclosure may preferably lead to a treated wound healing at a rate at least 5% faster than an untreated wound, preferably at a rate at least 10% faster, more preferably at least 15%, 20% or 25% faster; yet more preferably at least 50% faster, still more preferably at least 75% faster, and most preferably 100% (or more) faster.

Accelerated healing of a wound in accordance with the present disclosure may preferably lead to a treated wound having a "healing age" that is at least a day faster than an untreated wound, preferably at a rate at least five days faster, more preferably at least ten days faster; yet more preferably at least eleven, twelve, thirteen, fourteen of fifteen days faster, still more preferably fifteen or more days faster, and most preferably 20 (or more) days faster. The skilled person will appreciate that acceleration of healing by even one day represents a clinically significant result, as reflected in guidance of clinical significance provided by the FDA.

### "Treated wounds", "control-treated wounds" and "untreated wounds"

A "treated wound" in the context of the present disclosure is any wound that has been provided with a therapeutically effective amount of a medicament of the invention.

"Control-treated wounds" and "untreated wounds" in the present context are respectively wounds treated with a relevant control, and wounds that have not been treated before, or during, healing. Control wounds will not be treated with a medicament of the invention, and preferably will not be treated with a therapeutically effective amount of an active compound. That said, wounds treated with medicaments known from the prior art may constitute suitable control wounds for comparative purposes (for example to illustrate increased efficiency or effectiveness of medicaments of the invention as compared to those already known).

The skilled person will appreciate that suitable control wounds should preferably be selected such that they are "matched" with the treated wounds, having reference to parameters such as the wound type, wound size, subject age, and subject health status.

It will be appreciated that untreated wounds will be expected to have a rate of healing that is not accelerated, while control wounds may be subject to either accelerated or retarded healing depending on the activity of the control administered.

### "Centimetre of wound" and "inch of wound"

A "wound centimetre", "centimetre of wound" or "centimetre of wounding" in the context of the present disclosure constitutes a unit by which the size of a wound to be treated may be measured.

A wound centimetre may be taken to comprise any square centimetre of a body surface that is wounded in whole or in part. For example, a wound of two centimetres length and one centimetre width (i.e. with a total surface area of two centimetres²) will also be considered to constitute "two wound centimetres", while a wound having a length of two centimetres and a width of two centimetres (i.e. a total surface area of four centimetres²) will constitute four wound centimetres. By the same token, a linear wound of two centimetres length, but of negligible width (i.e. with negligible surface area), will, for the purposes of the present invention, be considered to constitute "two wound centimetres", if it passes through two square centimetres of the body surface.

The size of a wound in wound centimetres should generally be assessed when the wound is in its relaxed state (i.e. when the body site bearing the wounded area is in the position adopted when the body is at rest). In the case of skin wounds, the size of the wound should be assessed when the skin is not subject to external tension.

An inch of wound may be similarly defined, save that the relevant units of length or area are measured in inches rather than centimetres.

A centimetre or inch of wounding may thus provide a unit by which the size of a wound to be treated may be measured, and the required amount of a medicament of the invention may be determined.

As noted above, the skin suffers from more direct, frequent, and damaging encounters with the external environment than any other organ in the body. As a result the skin suffers from more wounds than other organs, and it is therefore highly desirable to be able to accelerate the healing of skin wounds in order to return this organ, as rapidly as possible, to its maximum functional effectiveness. Acceleration of the healing of skin wounds is a preferred embodiment of the medicaments of the present invention. Skin wounds susceptible to accelerated healing in accordance with the medicaments of the invention include both "open" wounds, in which the integrity of the skin has been entirely compromised, exposing the underlying tissues, and also "closed" wounds in which the skin, though damaged, is not entirely compromised. Martial thickness wounds, as described elsewhere in the specification (particularly in the context of dermal peels, chemical peels, skin grafts), provide an example of closed wounds that may benefit from accelerated healing as provided by the invention.

Accelerating the healing of skin wounds in accordance with the invention is able to hasten the formation of a functioning protective barrier over previously damaged or denuded areas. The accelerated healing helps prevent ingress into, and colonisation of, the underlying tissue by pathogens such as bacteria, fungi and viruses. Thus accelerating the healing of wounds using the medicaments of the invention may provide benefits in contexts in which it is desirable to prevent or reduce infection of wounds.

Healed skin (and in particular the re-established intact epidermis) also acts as a barrier to fluid movement, and is therefore able to prevent desiccation of underlying tissue. Thus accelerated wound healing that may be achieved with the medicaments of the invention may help to prevent or reduce tissue desiccation arising as a result of fluid loss across damaged areas of the skin. As set out elsewhere in the specification, the damage caused as a result of fluid loss through wounded skin is particularly damaging in the case of wounds having large surface areas, such as burns. The acceleration of the healing of bums wounds thus represents a preferred application of the methods or medicaments of the invention.

It will be appreciated that accelerated healing of wounds that may be achieved by the medicaments of the invention may be of particular benefit in cases in which the wound healing response is impaired, inhibited, retarded or otherwise defective as compared to the normal rate of healing. The medicaments of the invention may also be used to accelerate the healing of wounds in patients that are not subject to an impaired healing response. Illustrative examples of both contexts are set out below.

There are many contexts in which the body's healing response is defective and may benefit from acceleration using the medicaments of the invention. These include conditions such as pemphigus, Hailey-Hailey disease (familial benign pemphigus), toxic epidermal necrolysis (TEN)/Lyell's syndrome, epidermolysis bullosa, cutaneous leishmaniasis and actinic keratosis. Retarded healing of wounds of the eye may be associated with conditions such as partial limbal stem cell deficiency or cornea erosions.

Healing of wounds may also be retarded as a result of the actions of pathogens (such as bacteria, fungi or viruses), chemical insults (such as chemical bums caused by caustic agents, or through the effect of cytotoxic drugs such as those employed in chemotherapy), or as a result of radiation damage (either through particulate radiation or electromagnetic radiation such as gamma radiation, ultraviolet radiation, or the like) such as that occurring in sunburn. Accordingly wounds subject to any of these influences may be particularly suitable subjects for acceleration of healing using the medicaments of the invention.

It is well known that dermal injuries in the aged heal more slowly than do those of younger individuals. The aged may therefore particularly benefit from accelerated healing brought about by the medicaments of the invention. There are also many other conditions or disorders that are associated with a delayed or otherwise impaired wound healing response. For example patients with diabetes, patients with polypharmacy (for example as a result of old age), post-menopausal women, patients susceptible to pressure injuries (for example paraplegics), patients with venous disease, clinically obese patients, patients receiving chemotherapy, patients receiving radiotherapy, patients receiving steroid treatment or immuno-compromised patients may all suffer from impaired epithelial regeneration. In some cases the slower healing response exhibited by such patients may contribute to the development of infections at the site of wounds. The slow wound healing response may also be associated with the formation of chronic wounds, as considered below. Accordingly, it will be appreciated that such patients represent a preferred group that may benefit from accelerated wound healing using the medicaments of the invention.

The inventors believe that the medicaments of the invention may be of particular value to aged or senescent patients. Aged or senescent patients, for the purposes of the present disclosure, may be defined as comprising patients aged 65 years or older, more preferably aged 75 years or older. In the case of female patients, it may be preferred that the medicaments of the invention be provided to post-menopausal patients, to whom they may be of marked benefit.

Without detracting from the above, it may generally be preferred that the medicaments of the invention may be utilised to accelerate the healing of wounds of patients not subject to delayed wound healing. Acceleration in this way will give rise to a faster wound healing response than would normally be achieved by such patients in the absence of therapeutic acceleration (i.e. give rise to faster healing than in control wounds). Accordingly the wounds of such patients may be induced to heal more rapidly.

The skilled person will immediately appreciate that there is a great benefit to be gained by society from the development of therapeutic agents and techniques that can hasten the healing of otherwise healthy patients. As well as the various benefits considered elsewhere in the specification, accelerating healing in this manner can help reduce time spent in convalescence, and can thus benefit productivity. Accordingly, the acceleration of healing of wounds of healthy patients is a preferred embodiment of all aspects of the present invention.

The medicaments of the invention may be used to accelerate the healing of both chronic wounds and acute wounds. For the purposes of the present invention, a chronic wound may be defined as any wound that does not show any healing tendency within eight weeks of formation when subject to appropriate (conventional) therapeutic treatment. Acute wounds may be any wound other than a chronic wound.

Acceleration of the healing of chronic wounds is a preferred embodiment of the invention. Examples of chronic wounds that may benefit from accelerated healing provided by the medicaments of the invention may be selected from the group comprising: leg ulcers; venous ulcers; diabetic ulcers; bed sores; decubitus ulcers; foot ulcers; and pressure ulcers. It will be appreciated that the long lasting nature of chronic wounds exacerbates many of the disadvantages associated with normal wound healing. For example, the duration of the period over which a patient suffering from a chronic wound will experience pain will generally be far longer than for a patient with an acute wound. Similarly the length of time over which desiccation as a result of liquid loss may occur will also be extended. Incidences of wound infection are also much increased in chronic, as opposed to acute, wounds.

Chronic wounds are also subject to many disadvantages that are not generally associated with acute wounds. For example, chronic wounds frequently expand beyond the limits of the original wounded area. This may arise as a result of infection (which may increase the damage around the margins of the wound, thereby leading to expansion) or through maceration of the tissue surrounding the wound (typically as a consequence of increased liquid loss through the chronic wound). The propensity for chronic wounds to expand beyond the boundary of the original injury means that such wounds are frequently of great surface area. Since accelerated wound healing utilising the medicaments of the invention occurs from "the outside in", it will be appreciated that this will act to counter the progression of chronic wounds, use of the medicaments of the invention may be of notable benefit in the treatment of chronic wounds.

Pretibial lacerations are acute wounds of the leg that are very frequently slow to heal, and which frequently give rise to the development of leg ulcers. Existing treatments used for pretibial lacerations include the use of surgical procedures (such as the use of skin grafts and flaps) in an attempt to heal the wound before chronic wound development. Pretibial lacerations constitute acute wounds that may particularly benefit from treatment with the medicaments of the invention, in order to accelerate healing and thereby reduce incidences of chronic wound formation.

The promotion of re-epithelialisation of acute wounds (as opposed to chronic wounds) is also a preferred embodiment of all aspects of the invention: Acute wounds, the healing of which may be accelerated using the medicaments of the invention, include: abrasions; avulsions; crush wounds; incisional wounds; lacerations; punctures; and missile wounds, all of which may be suffered by the skin (among other tissues or organs).

Abrasions are also commonly referred to as "scrapes". Abrasions occur as a result of the skin being rubbed away by friction against another rough surface. Common examples of abrasions include rope burns and skinned knees. An abrasion may macroscopically appear as lines of scraped skin, possibly including tiny spots of bleeding.

Avulsions occur when an entire bodily structure, or a part of such a structure, is forcibly pulled away from its site. Examples of avulsions include the loss of a permanent tooth or an ear lobe. Avulsions may, for example, arise as a result of explosions, gunshots, and animal bites. An avulsion may characteristically exhibit heavy, rapid bleeding, as well as a noticeable absence of tissue.

Crush wounds typically occur as a result of a heavy object falling onto an individual (or part of an individual). The force thus generated may split the skin and shatter or tear underlying structures. A crush wound may have irregular margins, similar in appearance to those of a laceration; however, the wound will generally be deeper and trauma to underlying muscle and bone may be apparent Incisional wounds are also commonly referred to as "cuts". Incisional wounds result from incision, or slicing, of a tissue with a sharp instrument, which results in a wound with relatively even edges. Incisional wounds can vary greatly in their severity, from minimal wounds (such as a paper cut) to significant wounds such as those arising as a result of surgical incision. An incisional wound may have little or profuse bleeding depending on the depth and length of the wound, and also on the tissue involved. The even edges of incisional wounds will generally readily line up, which may facilitate closure of such wounds.

Lacerations are also frequently referred to a "tears". These wounds arise as a result of forcible separation of a tissue or organ, which will normally produce a wound having characteristic ragged edges. Lacerations are generally produced by the action of great mechanical forces against the body, either from an internal source as in childbirth, or from an external source like a punch. The laceration arises when the force exerted on a tissue or organ becomes too great for the tissue or organ to bear. A laceration may exhibit little or profuse bleeding, in much the same manner as an incisional wound. In contrast to incisional wounds however, the tissue damage is generally greater and the wound's ragged edges do not line up so readily.

Punctures are deep, narrow wounds. Punctures may typically be produced by sharp objects such as nails, knives, and broken glass being driven into the body. The depth of a puncture wound will generally be greater than its length. As a consequence there is generally little bleeding around the outside of the wound although more bleeding may occur inside the wound. This may lead to discoloration around the puncture wound.

Missile wounds are also known as "velocity wounds". Missile wounds are caused by an object entering the body at a high speed, typically a bullet. A missile entry wound may be accompanied by an exit wound, and bleeding may be profuse, depending on the nature of the injury.

Incisional wounds constitute preferred acute wounds healing of which may be accelerated by the medicaments of the invention. Surgical incisional wounds may constitute a particularly preferred group of acute wounds to be treated in accordance with the invention.

It will be appreciated that tissues other than the skin, such as the cornea, may also be subject to wounds of the type described above and elsewhere in the specification. Such wounds may also benefit from the acceleration of healing that is provided by use of medicaments of the invention.

Burn wounds are a further class of wounds the healing of which may be accelerated using the medicaments of the invention. For the purposes of the present disclosure, bums may, except for where the context requires otherwise, be considered to include tissue damage resulting from exposure to either high or low temperature, chemical agents, or radiation.

Wounds arising as a result of bums may extend over great areas of an individual so afflicted. As a result bum wounds are particularly susceptible to complications such as infection and desiccation. It will be appreciated that bums wounds may therefore derive particular benefit from the accelerated wound healing that may be brought about by the medicaments of the invention.

The use of the medicaments of the invention to accelerate the healing of wounds associated with skin grafting procedures represents a preferred embodiment of the invention. It is a preferred embodiment of the medicaments of the invention to accelerate "normal" rates of healing of wounds associated with grafting procedures (i.e. to accelerate the healing of grafting wounds in patients without an impaired healing response).

It is another preferred aspect of the invention to accelerate the healing of grafting wounds in patients subject to impaired or reduced healing responses. Grafting may frequently be used in an attempt to promote the healing of other wounds (particularly chronic wounds) in patients subject to impaired healing, and it may be desired to accelerate the healing of wounds associated with the graft in order to reduce the likelihood of these developing adverse complications.

The accelerated healing conferred by the medicaments of the invention is of benefit at the graft recipient site, and at the graft donor site. The medicaments of the invention may be used to accelerate healing of wounds associated with both full and partial thickness skin grafts. Such skin grafts (i.e. either full or partial thickness grafts) may be either meshed or unmeshed.

At the graft recipient site the accelerated healing provided by the medicaments of the invention is able to improve and accelerate integration of the grafted tissue. Since the beneficial effects of the medicaments of the invention are believed to come about as a result of effects on the unwounded tissue surrounding the wounded site it will be appreciate that these advantageous effects may be available not only in the case of grafts utilising skin, but also in the case of grafts using artificial skin, or skin substitutes (since keratinocytes from the surrounding unwounded skin may be induced to migrate into the wounded area and thereby encourage integration of the graft).

The acceleration of wound healing of graft donor sites decreases the time taken to restore a functioning skin barrier layer, and consequently reduces the potential for donor site infection. The accelerated healing also decreases incidences of blistering and tissue breakdown that may otherwise occur at the donor site.

Since wounds treated in accordance with the present invention heal faster the period over which a patient experiences pain associated with sites where the skin has been damaged or removed is reduced. Thus by accelerating the healing of wounds at such sites using the medicaments of the invention it is possible to reduce the pain associated with the taking of skin grafts.

A further advantage of accelerated healing of wounds at skin donor sites is that this decreases the time required until re-harvesting of tissue from the donor site can take place. By "re-harvesting" is meant the subsequent removal of further graftable skin from a previously used donor site. This is particularly advantageous in situations where the skin available for harvesting is limited and/or the area of skin required to be harvested is large. Examples of such situations include occasions when it is necessary to take grafts from children and/or patients suffering from bums covering a large percentage of the body surface. In these cases there may be relatively little unwounded skin that may serve as a donor site for graft material, and it may be desirable to obtain multiple donations from the sites available.

Graft donor sites may generally be treated with the medicaments of the invention before the graft tissue is taken. This allows the donor site to be "primed" in advance of wounding. Such priming may further help to accelerate wound healing by avoiding any "lag" that may otherwise occur between the graft being taken and a medicament administered. Prophylactic use in this manner may further help to avoid onset of complications that may otherwise arise at the graft donor site, such as chronic wound development.

It will be appreciated that the medicaments of the invention may be used prophylactically in situations other than grafting procedures, such as before surgery or when there is a risk of a wound occurring through other means. Prophylactic use in this manner may be of benefit to both healthy and healing-impaired patients. In the case where the individual to be wounded may otherwise be subject to retarded or incomplete wound healing it will generally be preferred that the medicaments of the invention are administered as soon as the risk of a poor rate of wound healing has been recognised.

Accelerated healing, such as that which may be brought about using the medicaments of the invention, may also give rise to a treated wound that has increased mechanical strength as compared to a control or untreated wound. This ability to increase the strength of wounds treated using the medicaments, of the invention, confers a notable advantage over control or untreated wounds.

The inventors have found that accelerated healing of wounds in accordance with the invention may be brought about using all oestrogens tested to date. The inventors believe that suitable oestrogens may be selected from the group consisting of:
ethinylyoestradiol, dienoestrol, mestranol, oestradiol, 17β-oestradiol, oestriol, conjugated oestrogens, and phytoestrogens.

As set out above, 17β-oestradiol constitutes a preferred oestrogen to be used in the medicaments of the invention. However, the inventors believe that the benefits provided by the medicaments of the invention are applicable to all oestrogens that may be able to therapeutically promote oestrogenic activity.

A preferred medicament in accordance with the present invention may comprise a maximum of 1% by weight of the oestrogen (such as 17β-oestradiol). More preferably, a medicament of the invention may comprise a maximum of 0.1 % by weight of the oestrogen (such as 17β-oestradiol), and even more preferably a medicament of the invention may comprise a maximum of 0.01% by weight of the oestrogen (such as 17β-oestradiol).

A suitable daily dose of an oestrogen will depend upon a number of factors, including the size of the wound to be treated. The influence of wound size on suitable therapeutic amounts of an oestrogen is considered in greater detail below. Typically the amount of an oestrogen that will be required for the treatment of wounds or fibrotic disorders will be within the range of 1 ng to 100g of the active compound in a given 24 hour period, depending upon the size of the wound healing of which is to be accelerated, and the identity of the active compound selected.

It may be preferred that a medicament in accordance with any aspect of the present invention be one in which the medicament provides an amount of oestrogenic activity equivalent to that produced by a tissue concentration of between 1µM and 10µM of 17β-oestradiol, more preferably by a tissue concentration of between 2µM and 8µM of 17β-oestradiol, even more preferably by a tissue concentration of between 3µM and 7µM of 17β-oestradiol. Suitable medicaments may provide equivalent oestrogenic activity to that provided by a tissue concentration of 3.3µM of 17β-oestradiol or a tissue concentration of 6.6pM of 17β-oestradiol. The inventors have surprisingly found that medicaments capable of providing such low amounts of oestrogenic activity remain capable of accelerating wound healing, and indeed do so more effectively than compositions that provide larger quantities of oestrogenic activity.

Indeed, the inventors have found that medicaments of the invention that comprise as little as 0.0001% by weight of an oestrogen (such as 17β-oestradiol) may be used to effectively accelerate wound healing. This proportion is markedly lower than the amounts previously considered in the prior art, where it was suggested that a suitable composition may preferably comprise approximately 1% by weight of an active compound.

The use of medicaments of the invention that administer between 0.02 µg and 0.3 µg (and preferably between 0.1 µg and 0.2µg) of an active compound such as 17β-oestradiol per wound centimetre represents a surprisingly effective sub-range within the broader range disclosed in the prior art (where amounts of between 0.005µg and 4µg of 17β-oestradiol are considered for administration per wound centimetre). There is nothing in the prior art that would have led the skilled person to consider the selection of this sub-range, and certainly nothing that would lead the skilled person to believe that this particular sub-range would be more effective than the ranges disclosed in relation to medicaments described in the prior art.

Thus it will be seen that medicaments in accordance with this embodiment of the invention provide notable advantages over the prior art. Not only are the medicaments more effective than the prior art medicaments (i.e. capable of inducing a greater acceleration of wound healing than may be achieved using medicaments known from the prior art), but they are also cheaper to manufacture than prior art compositions, since they incorporate less of the oestrogens.

Preferably the acceleration of wound healing using the medicaments of the invention may give rise to a healing time 1 day, 2 days, or 3 days faster than that occurring in a control-treated or untreated wound. Healing time may be calculated as the time elapsing between formation of a wound and complete re-epithelialisation of the wound. More preferably accelerated healing in accordance with the invention may give rise to a time to a healing time that is at least 4 days, 5 days or 6 days faster than that occurring in a control-treated or untreated wound. It is even more preferred that accelerated healing may give rise to a time to a healing time that is at least 7 days, 8 days or 9 days faster than that occurring in a control-treated or untreated wound, and most preferably accelerated healing may give rise to a time to re-epithelialise that is at least 10 days or greater than that occurring in a control-treated or untreated epithelium.

With respect to reducing the time that must elapse to allow re-harvesting of graft material from skin donor sites, preferably the acceleration of healing may give rise to a time to re-harvesting that is 1 day, 2 days, or 3 days faster than that occurring in a control-treated or untreated graft donor sites. More preferably acceleration of the healing of wounds using the medicaments of the invention may give rise to a time to re-harvesting that is at least 4 days, 5 days or 6 days faster than that occurring in a control-treated or untreated graft donor site. It is even more preferred that accelerated healing of wounds may give rise to a time to re-harvesting that is at least 7 days, 8 days or 9 days faster than that occurring in a control-treated or untreated graft donor site, and most preferably accelerated healing of wounds may give rise to a time to re-harvesting that is at least 10 days or greater than that occurring in a control-treated or untreated graft donor site.

The inventors have found that the medicaments of the invention are able to accelerate healing of wounds when used either prior to the formation of a wound, or when used after a wound has already been formed. The use of the medicaments of the invention prior to formation of a wound (in which case it is believed that the action of the medicament is to "prime" the site where wounding will occur so that the healing response is accelerated immediately upon formation of the wound) is referred to as "prophylactic use" for the purposes of the present disclosure.

The prophylactic use of agents in accordance with the invention to accelerate the healing of wounds is a preferred mode of use in accordance with the invention. It will be appreciated that such use is most suitable in the case where the time and location of prospective wound formation is known, and may be particularly suitable for accelerating the healing of wounds associated with surgical procedures. However, prophylactic use of the medicaments of the invention may also be of use in situations where there is an increased likelihood of wounding occurring. The inventors have found that administration of agents in accordance with the invention immediately prior to formation of a wound (e.g. in the hour, or preferably half hour, or more preferably ten minutes, preceding wounding) is highly effective, though administration at earlier times (e.g. up to 24 or 48 hours before wounding) may also beneficially accelerate the healing of wounds. The prophylactic use of medicaments of the invention is a preferred embodiment of the invention, and is particularly preferred for accelerating the healing of skin graft donor and/or recipient sites.

Injection, and particularly intradermal injection, constitutes a preferred manner in which the medicaments of the invention may be administered, as considered elsewhere in the specification. In the case of prophylactic use, it may be particularly preferred that a medicament of the invention be administered by intradermal injection to a site where wounding will take place. If the medicament is administered only a short time prior to wound, then intradermal injection of this type will typically lead to the formation of a raised bleb which will remain at the time of wounding. A wound may then be formed through the bleb. Wounds formed in this way will benefit from accelerated healing in accordance with the present invention. Alternatively, blebs formed by intradermal injection of medicaments of the invention may be allowed to resolve before a wound is formed.

The medicaments of the invention may also be used to accelerate the healing of wounds once the wound in question has already been formed. This use will be the use generally adopted in respect of accidental wounds (and indeed most wounds formed other than in association with a surgical procedure, although even these wounds may be effectively treated after their formation).

When used to treat existing wounds medicaments in accordance with the invention may preferably be injected along the margins of wounds to be treated. In the case of skin wounds it is preferred that the route of injection selected is intradermal injection.

In the event that the medicaments of the invention are to be used to accelerate the healing of an existing wound, it is preferred that such use should occur as early as possible after formation of the wound. That said, the medicaments of the invention may help to accelerate healing of a wound if used at any time up until full healing has occurred (for example, even if administered to a partially healed wound the medicaments of the invention may accelerate the healing in respect of that portion of the wound that remains as yet unhealed).

Factors that may be considered in relation to the "window" in which medicaments of the invention may be beneficially employed such that they are able to accelerate the healing of wounds will include: the nature of the wound in question (for example: is the wound at a site that is generally subject to "fast" or "slow" healing?); the severity of the wound (what is the extent of the damage that has occurred?); and the size of the damaged area. Thus in the case of a wound of large area, or in a site that is naturally associated with slower than average healing, the medicaments of the invention may be still be effective to accelerate the healing of the wound even if administered relatively late in the healing response. Thus, although the medicaments of the invention may preferably be administered within the first one to 24 hours after formation of an acute wound, beneficial acceleration of healing may also be brought about if administered up to ten, or more, days after the wound is formed.

It will be appreciated that in the case of chronic wounds, the period in which the medicaments of the invention may be beneficially employed will be considerably longer. Chronic wounds may persist for many years, and the healing of wounds that may be many years old may be beneficially accelerated using the medicaments of the invention.

Acceleration of the healing of wounds may be achieved using only a single administration of the medicaments of the invention. Due to the simplicity of this therapeutic regime it constitutes a preferred use of the medicaments of the invention. Thus a medicament of the invention may be formulated such that a therapeutically effective amount of an oestrogen is provided a single administered dose.

However, there may be cases in which it is preferred that the medicaments of the invention be used in repeated incidences of therapy. Thus treatment to accelerate healing of a wound may involve administration of medicaments of the invention on more than once occasion. Use in this manner may be preferred in the case of large wounds, or of wounds that are resistant to treatment, or subject to retarded healing (such as chronic wounds). Generally medicaments of the invention may be administered to a wound as required until healing has been achieved. By way of example medicaments of the invention may be administered daily (or on multiple occasions within a given day), or may be administered after a delay of multiple days.

Generally when the medicaments of the invention are to be used in multiple therapeutic incidences administration should be repeated until acceleration of healing has been achieved to a clinician's satisfaction.

It may be preferred that the medicaments of the invention are utilised both before and after wounding.

The medicaments of the invention are preferably administered topically (i.e. administered to an existing wound or to a site where a wound will be formed). Although the present disclosure has primarily related to the use of medicaments of the invention in injections, it will be appreciated that medicaments of the invention may be used topically on the surface of areas to be treated (such as wounds or sites where wounds are to be formed) to achieve the same therapeutic effects. By way of example, the inventors believe that medicaments of the invention may be used therapeutically as irrigation fluids. The inventors believe that medicaments of the invention according to all aspects and embodiments disclosed herein (except for where the context requires otherwise) may be employed in such uses.

Administration by injection represents a preferred method by which medicaments of the invention may be topically administered.

As has been set out previously, it is a preferred embodiment of the invention that the medicament is an injectable medicament. Suitable formulations for use in this embodiment of the invention are considered below, and are also set out in the Experimental Results section.

Medicaments of the invention, such as irrigation fluids of the invention, may additionally comprise one or more agents independently selected from the group consisting of: cleansers; antibiotics; antifungal agents; antiseptic agents; and anaesthetic agents. Medicaments of this sort may be of particular value in the treatment of cavitating and/or chronic wounds.

Medicaments of the invention comprise a phosphate buffer, which under the majority of circumstances in which the medicaments may be used will serve to buffer the medicament. With this aim in mind, it will be appreciated that the amount of a phosphate buffer provided in a medicament of the invention should generally be sufficient to allow buffering of the medicament within physiologically acceptable limits. The use of different phosphate components, and of slight changes in the ratios of phosphate components, subtly alter the pH of the buffer mixture are well documented in the literature. Suitable combinations of phosphate components that may be used to achieve formulations having required pH values will be well known to those skilled in the art.

Typically a phosphate buffer suitable for use in the medicaments of the invention may comprise potassium dihydrogen phosphate in combination with disodium phosphate. In the case of medicaments of the invention utilising this combination, a suitable concentration of potassium dihydrogen phosphate to be incorporated may be between 0.1 mM and 20 mM., preferably between 1 mM and 2 mM, more preferably between about 1.2 mM and 1.5 mM, even more preferably between about 1.20mM and 1.54mM (inclusive), and most preferably about 1.5 mM.

An amount of disodium phosphate that may be used in a medicament of the invention may be between 0.1 mM and 20 mM. A more preferred concentration may be between about 2.5 and 3.5 mM, even more preferably the concentration may be between about 2.70 mM, and 3.06 mM (inclusive) and most preferably about 2.7 mM.

The total phosphate content of a medicament of the invention may be between 0.1 mM and 50 mM and is most preferably approximately 4.2 mM.

pH in the body can vary from site to site and it is the inventors' belief that the enhanced buffering capabilities provided by the phosphate buffering system increase the biological activity of the oestrogen , and thereby increases the therapeutic effectiveness of the medicaments of the invention.

Since the medicaments of the invention are generally to be formulated for administration by injection, their pH may preferably be controlled such that it lies within a range of values that will not adversely affect tissue at the site, or sites, at which the medicaments are to be administered. Control of the pH of the medicaments may preferably be exerted via the phosphate buffer used in the medicaments. Although the medicaments may vary between physiologically acceptable limits, it is preferred that medicaments of the invention have a pH of between 6.7 and 7.7. It is particularly preferred that the pH of a medicament of the invention is approximately 7.2.

The medicaments of the invention comprise an alcohol. This alcohol may serve as a solvent for the oestrogen, or may aid the solubility of the compound in other solvents used in the medicament. The inventors have found that the medicaments of the invention are most effective when they contain an alcohol at a concentration of between 0.17M and 1.71M. The concentration of the alcohol in a medicament of the invention is most preferably approximately 0.85M.

Various different alcohols may be used in the medicaments of the invention. Alcohols that may be utilised in the medicaments of the invention should be capable of contributing to the solubility of compounds utilised in the medicaments, and particularly to the solubility of the oestrogen (such as 17β-oestradiol). The inventors have found that the use of alcohols, such as ethanol, to promote solubility of such compounds is far preferable to the use of alternative agents described in the prior art. These include cyclodextrins and other agents such as arachis oil, castor oil, TWEEN, sugars as exemplified by mannose. The inventors have found that agents, such as cyclodextrins, that have been used in wound healing compositions of the prior art may serve to impair the wound healing response. Thus although prior art compositions comprising oestrogen solubilised in the presence of cyclodextrins may serve to increase the rate of wound healing their activity is not as efficient as the medicaments of the present invention, since at least some of the beneficial activity elicited by the oestrogenic compound is counteracted by the unwanted effects of cyclodextrins.

It is also generally desirable that medicaments of the invention make use of diluents that are substantially free from allergens or toxins, such as those that may be found in naturally occurring products (such as nut or vegetable oils). It will be appreciated that the considerations above are of particular concern in the case of medicaments of the invention in which oestrogens are administered by means of injection. Furthermore, the inventors have found that the use of oil-based diluents in the preparation of compositions for administration by localised injection (such as intradermal injection) to wounds, or sites where wounds are to be formed, may generally be disadvantageous, and can give rise to deleterious effects that may retard the wound healing process and lead to the production of abnormally wide wounds. Thus it may be preferred not to use oils in the preparation of injectable medicaments in accordance with the invention.

It is preferred that the alcohol is selected from the group consisting of: ethanol and higher water miscible alcohols known in the art as solubilisers/excipients. Medicaments of the invention may comprise more than one alcohol selected from the list set out above. Ethanol represents a preferred alcohol to be used in the medicaments of the invention (either alone, or in combination with another alcohol).

Medicaments of the invention suitable for injection should preferably be approximately isotonic with the tissue into which they are to be injected. One of the major factors in determining tonicity of injectable solutions is the concentration of chloride ions present in the solution. In order that a medicament of the invention may have a tonicity that renders it suitable for injection, it may be preferred that the medicament comprises a source of chloride ions. Suitable sources of chloride ions include sodium chloride and/or potassium chloride. Sodium chloride represents a preferred source of chloride ions for use in the medicaments of the invention.

In the case that a medicament of the invention comprises sodium chloride, it may be preferred that the concentration of sodium chloride is between 130 mM and 180 mM. It may be particularly preferred that the concentration of sodium chloride is approximately 154 mM.

The amounts of sodium chloride and potassium chloride described above are particularly suitable for use in medicaments where both sodium chloride and potassium chloride are present.

It will be appreciated that medicaments of the invention are suitable for preparation in various forms, depending on the preferred manner in which they are intended to be used.

Purely by way of example, it will be appreciated that medicaments of the invention may be provided in a concentrated form, which may be diluted using appropriate diluent to generate a "working strength" solution to be administered to a patient in order to accelerate healing of a wound.

Although it is preferred that the medicaments of the invention be used in human patients, it will be appreciated that many of the advantages that may be gained as a result of accelerated healing of human wounds are also are also applicable to healing of wounds in other animals, particularly veterinary or domestic animals (e.g. horses, cattle, dogs, cats etc). Accordingly it will be recognised that the medicaments of the invention may also be used to accelerate the healing of wounds of non-human animals.

The invention will now be further described with reference to the accompanying Experimental Results and Figures, which illustrate the surprising effectiveness of medicaments of the invention.
Figure 1 shows that a medicament of the invention accelerates the healing of full thickness cutaneous excisional wounds (punch biopsies) in male rats compared to cyclodextrin formulation.
Figure 2 shows that a medicament of the invention accelerates the healing of full thickness cutaneous excisional wounds (punch biopsies) in male rats compared to saline formulation.
Figure 3 shows a comparison of therapeutically effective amounts of an oestrogen incorporated in a medicament of the invention with therapeutically effective amounts suggested by the prior art.
Figure 4 shows acceleration of healing of partial thickness wounds using medicaments of the invention.
Figure 5 shows that a medicament of the invention accelerates the healing of full thickness cutaneous excisional wounds (punch biopsies) in humans.

### EXPERIMENTAL RESULTS

### 1 Comparison of medicaments of the invention with medicaments of the prior art

The following study was undertaken to compare acceleration of re-epithelialisation, and hence wound healing, brought about using the medicaments of the invention, with the level of healing brought about using medicaments disclosed in the prior art. The model of wound healing involved utilised investigation of full thickness excisional wounds in experimental rats.

The prior art medicament investigated comprised a solution of 17β-oestradiol (encapsulated in cyclodextrin) at a concentration of 3.67mM (corresponding to a 0.1% solution 17β-oestradiol of the type described in the prior art), thus providing 100µg of 17β-oestradiol per 100µl injection.

In contrast, the medicament of the invention comprised 17β-oestradiol at a concentration of 3.67µM in a solution comprising an alcohol and a phosphate buffer thus providing 0.1 µg of 17β-oestradiol per 100µl injection. As set out elsewhere in the specification, the surprisingly low amounts of oestrogen that may be used therapeutically effectively in the medicaments of the invention provide a considerable advantage over the medicaments described in the prior art.

### 1.1 Materials and methods

### 1.1.1 Medicament of the invention

An injectable medicament in accordance with the present invention was prepared by the incorporation of 17β-oestradiol in a solution comprising 95% phosphate buffered saline and 5% ethanol. Constituents of the injectable solution were as follows (amounts present in 1ml of injectable solution):

| | |
|---|---|
| 17β-oestradiol | 1µg |
| Ethanol | 50µl |
| KH₂PO₄ | 0.21mg |
| NaCl | 9.0mg |
| Na₂HPO₄.2H₂0 | 0.482mg |
| 0.1M NaOH | q.s. to pH 7.2 |
| 0.1M HCl | q.s. to pH 7.2 |
| Water for injection | to 1ml |

### 1.1.2 Prior art medicament

A 17β-oestradiol-containing medicament of a type described in the prior art for the acceleration of wound healing through promotion of re-epithelialisation was prepared. The constituents of this injectable medicament were as follows:

| | |
|---|---|
| 17β-oestradiol | 1mg |
| KH₂PO₄ | 0.21mg |
| NaCl | 9.0mg |
| Na₂HPO₄.2H₂0 | 0.482mg |
| 2-hydroxypropyl-β-cyclodextrin | 21.74 mg |
| 0.1M NaOH | q.s. to pH 7.2 |
| 0.1M HCl | q.s. to pH 7.2 |
| Water for injection | to 1ml |

### 1.1.3 Wound healing model

A rat excisional wound model was used to investigate the acceleration of re-epithelialisation, and hence wound healing, brought about by the medicaments tested.

100µl of the medicament to be tested was administered by intradermal injection at sites on the dorsal surface of experimental rats, 10 minutes prior to formation of a full thickness punch biopsy at the injected site. One injection and excisional wound was made per animal, and ten adult male Sprague-Dawley rats were used per treatment group.

### 1.1.4 Assessment of acceleration of wound healing

Animals were killed, and their wounds harvested, three days after wounding. The wounds were excised and treated for histology. Histological sections from wounds treated with medicaments of the invention and from wounds treated with prior art medicaments were studied and the percentage of re-epithelialisation exhibited by the different wounds calculated and compared. All histological assessments of early wound healing events were made using preserved 5 micron-thickness wound sections taken from the widest part of each excised biopsy site. Sections were stained with Haematoxylin and Eosin to aid visualisation of structural features, and measurements made using image analysis software. The distance travelled by the epithelium is derived from two histological measurements: the freehand total wound diameter and the freehand non-epithelialised wound diameter. The non-epithelialised diameter is subtracted from the total wound diameter, to determine the distance which the new epithelium has covered since the time of wounding. Percentage re-epithelialisation for the new epithelium was calculated as a proportion of the total wound diameter. The average percentage re-epithelialisation value was calculated from the wounds of the ten animals within each treatment group.

### 1.2 Results

Wounds treated with the prior art medicament exhibited 33.0% re-epithelialisation as assessed using the protocol described above.

In contrast, wounds treated with the medicament of the invention exhibited 35.7% re-epithelialisation as assessed using the protocol described above.

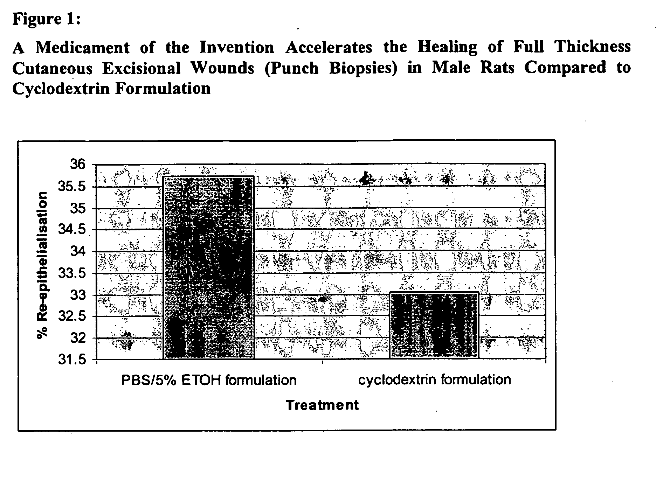

The results of the present study (shown in Figure 1) clearly indicate that the medicaments of the invention are able to accelerate wound healing (as demonstrated by an increase in the rate of re-epithelialisation) to a greater extent than medicaments described in the prior art, even though the medicaments of the invention incorporated a far lower concentration of 17β-oestradiol. It will be recognised that the ability to achieve greater acceleration of wound healing while using a smaller amount of an oestrogen confers surprising advantages over the prior art.

### 2 Comparison of medicaments of the invention and medicaments lacking a phosphate buffer

The inventors compared the degree of acceleration of wound healing obtained using medicaments of the invention with the degree of acceleration of wound healing attainable using control medicaments lacking a phosphate buffer. This study illustrates that the advantages demonstrated by the medicaments of the invention do not arise purely as a result of their lack of cyclodextrin (which is known to have some adverse effects *in vivo),* but also arise as a result of their inclusion of phosphate buffer.

In order to test this hypothesis the inventors compared acceleration of wound healing achieved on use of a medicament of the invention with that achieved on use of a comparable control medicament lacking a phosphate buffer. Both test medicaments incorporated 17β-oestradiol at a concentration of 3.67µM, a concentration shown to be effective in the context of medicaments of the invention in the study reported under heading 1. As with the previous study, the acceleration of wound healing was investigated in a rat full thickness excisional wound model.

### 2.1 Materials and methods

### 2.1.1 Medicament of the invention

The medicament of the invention was prepared as described in 1.1.1 above.

### 2.1.2 Control medicament

An injectable control medicament was prepared by the incorporation of 17β-oestradiol in a solution comprising 95% saline and 5% ethanol. Constituents of the injectable solution were as follows (amounts present in 1ml of injectable solution):

| | |
|---|---|
| 17β-oestradiol | 1µg |
| NaCl | 9.0mg |
| Ethanol | 50µl |
| 0.1M NaOH | q.s. to pH 7.2 |
| 0.1M HCl | q.s. to pH 7.2 |
| Water for injection | to 1ml |

### Wound healing model

The wound healing model used was as described in 1.1.3 above.

### Assessment of acceleration of wound bealing

Assessment of acceleration of wound healing was undertaken as described in 1.1.4 above.

### 2.2 Results

Wounds treated with the control medicament lacking phosphate buffer exhibited 33.0% re-epithelialisation as assessed using the protocol described above.

In contrast, wounds treated with the medicament of the invention exhibited 35.7% re-epithelialisation as assessed using the protocol described above.

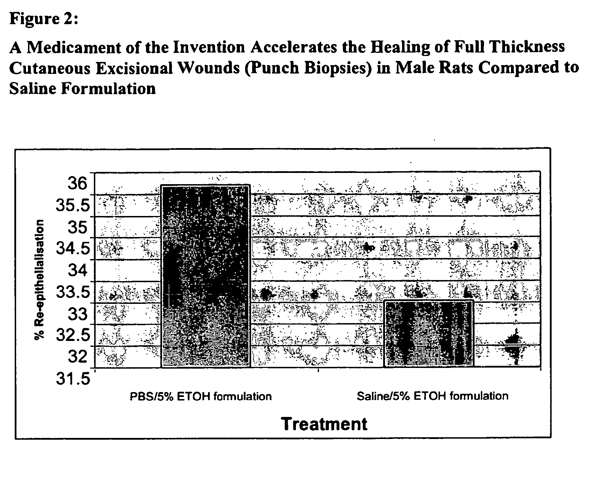

The results of this study (shown in Figure 2) clearly indicate that the medicaments of the invention are able to accelerate wound healing (as demonstrated by an increase in the rate of re-epithelialisation) to a greater extent than control medicaments that contain the same concentration of 17β-oestradiol, but lack a phosphate buffer. It will be recognised that the greater acceleration of wound healing achieved in wounds treated with the medicament of the invention serves to highlight the advantageous properties of these medicaments.

### 3 Comparison of therapeutically effective amounts of an oestrogen incorporated in a medicament of the invention with therapeutically effective amounts suggested by the prior art

The prior art has previously suggested that compounds that promote oestrogenic activity may be incorporated in compositions at concentrations of between 0.001% and 4% to promote accelerated wound healing (via an increase rate of re-epithelialisation). The prior art further suggests that compositions comprising such a promoter of oestrogenic activity at a concentration of between 0.01% and 2% by weight may be preferred compositions, and that compositions comprising a maximum of 1% 17β-oestradiol may be particularly preferred.

The inventors believe that medicaments in accordance with the present invention may achieve a greater acceleration of re-epithelialisation (and hence wound healing) than those described in the prior art, and may do so using much reduced amounts of an oestrogen. This ability to use a much-decreased amount of an oestrogen as a therapeutically effective amount able to accelerate wound healing to a greater extent than may be achieved in accordance with the prior art provides clear advantages.

### 3.1 Materials and methods

### 3.1.1 Preparation of medicaments of the invention

Medicaments of the invention were prepared, using the constituents described in 1.1.1 above, to produce medicaments of the invention comprising different test concentrations of 17β-oestradiol as follows:
i) 0.001µg 17β-oestradiol/100µl of medicament (0.000001% w/v)
ii) 0.01µg 17β-oestradiol/100µl of medicament (0.00001% w/v)
iii) 0.1µg 17β-oestradiol/100µl of medicament (0.0001 % w/v)
iv) 1µg 17β-oestradiol/100µl of medicament (0.001 % w/v)
v) 10µg 17β-oestradiol/100µl of medicament (0.01% w/v)

The test medicament comprising 1µg of 17β-oestradiol/100µl of medicament corresponded to the 0.001% solution that is the lowest therapeutically effective concentration suggested by the prior art, and the medicament comprising 10µg of 17β-oestradiol/100µl of medicament corresponded to the 0.01% solution that the prior art suggests as a preferred embodiment. Thus test medicaments i), ii) and iii) constituted solutions that were respectively 1000, 100 and 10 times less concentrated than the lowest therapeutically effective medicaments suggested by the prior art.

### 3.1.2 Wound healing model

A rat excisional wound model was used to investigate the acceleration of re-epithelialisation, and hence wound healing, brought about by the medicaments tested.

100µl of the medicament to be tested was administered by intradermal injection at sites on the dorsal surface of experimental rats, 20 minutes prior to formation of a full thickness punch biopsy at the injected site. One injection and excisional wound was made per animal, and five adult male Sprague-Dawley rats used per treatment group.

### 3.1.3 Assessment of acceleration of wound healing

Acceleration of wound healing achieved using the test concentrations of the medicaments of the invention was investigated using the protocols set out under 1.1.4 above.

### 3.2 Results

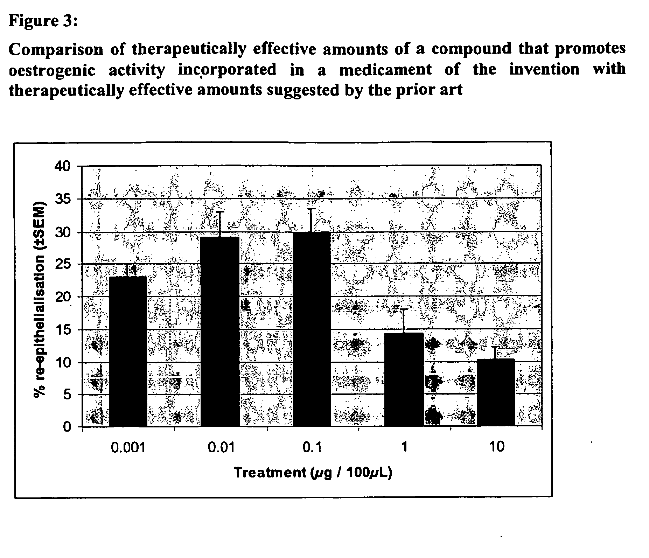

The results of this study are shown in Figure 3. As can be seen, re-epithelialisation was found in the case of all medicaments tested. Surprisingly, however, the results of this study illustrate that concentrations of 17β-oestradiol (an oestrogen) that are lower than the minimum therapeutically effective concentrations suggested by the prior art are actually able to accelerate re-epithelialisation (and hence wound healing) to a greater extent than the concentrations identified in the prior art.

Wounds treated with a medicament comprising 10µg 17β-oestradiol /100µl of medicament (corresponding to the preferred 0.01% solution suggested in the prior art) exhibited 10.30% re-epithelialisation, while wounds treated with a medicament comprising 1µg 17β-oestradiol/100µl of medicament (corresponding to the 0.001% solution identified by the prior art as the lowest therapeutically effective amount) exhibited 14.63% re-epithelialisation.

In contrast, wounds treated with a medicament comprising 0.1 µg 17β-oestradiol/100µl of medicament exhibited 29.95% re-epithelialisation, while wounds treated with a medicament comprising 0.01µg 17β-oestradiol/100µl of medicament exhibited 29.03% re-epithelialisation, and wounds treated with a medicament comprising 0.001µg 17β-oestradiol/100µl of medicament exhibited 23.02% re-epithelialisation, results which are all significantly higher than those obtained using the amounts of 17β-oestradiol suggested by the prior art.

This surprising activity of medicaments of the invention means that concentrations of an oestrogen up to 1000 times lower than those suggested by the prior art may be used to increase the rate of re-epithelialisation, and thereby accelerate wound healing. Furthermore, these concentrations are actually able to promote re-epithelialisation and accelerate healing to a greater extent than can the medicaments of the prior art. The advantages of the medicaments of the invention over the prior art are thus two-fold: the medicaments of the invention accelerate the healing of wounds to a greater extent than do the medicaments described in the prior art, and the medicaments of the invention are able to be produced more cheaply, efficiently and with lower risks of pollution than associated with prior art medicaments.

### 4 Acceleration of healing of partial thickness wounds using medicaments of the invention.

The study described here illustrates that medicaments of the invention are able to therapeutically effectively accelerate healing of partial thickness wounds (which provide a model of split thickness skin graft donor sites), and are able to do so when incorporating lower concentrations of an oestrogen than would be suggested by the teachings of the prior art.

### 4.1 Materials and methods

### 4.1.1 Medicaments of the invention

Medicaments of the invention incorporating a range of test concentrations of 17β-oestradiol (an oestrogen) were prepared using the constituents described above.

Three test concentrations of the medicaments were prepared as follows:
i) 0.1µg 17β-oestradiol/100µl of medicament (0.0001% w/v)
ii) 0.2µg 17β-oestradiol/100µl of medicament (0.0002% w/v)
iii) 0.4µg 17β-oestradiol/100µl of medicament (0.0004% w/v)

### 4.1.2 Wound healing model

A pig partial thickness wound healing model was used to assess the ability of the medicaments of the invention to accelerate the healing of wounds providing a model of split thickness skin graft donor sites.

Test medicaments were administered by intradermal injection to provide 100µl of medicament per cm² to sites on experimental pigs where wounds were to be formed. Thus a total volume of 625µl of the test medicament was administered per wound site. After administration of the medicaments 2.5cm by 2.5cm (6.25cm²) partial thickness wounds were made using a 25mm dermatome (Nouvag) at a depth setting of 0.55mm. A single wound was treated per animal, and each treatment group comprised four male Large White pigs.

### 4.1.3 Assessment of acceleration of wound healing

Animals were killed, and their wounds harvested, five days after wounding. The wounds were excised and treated for histology. Histological sections from wounds treated with the different medicaments of the invention were studied and the percentage of re-epithelialisation exhibited by the different wounds calculated and compared. After being allowed fixed for 48 hours wounded tissue was bisected into four even sections. All bisected sections were processed to wax and embedded into individual wax blocks. Tissue was sectioned through to the end of the block where at every 250µm, a single slide containing a 5µm thick section was prepared. Each section was stained with Haematoxylin and Eosin to aid visualisation of structural features, and measurements made using image analysis software. For each section, the freehand epithelialised and freehand non-epithelialised distances were measured using image analysis; freehand epithelialised distance as a proportion of the sum of the epithelialised and non-epithelialised distances was calculated to produce a percentage re-epithelialisation value. Average percentage re-epithelialisation values were calculated per wound and per treatment group.

### 4.2 Results

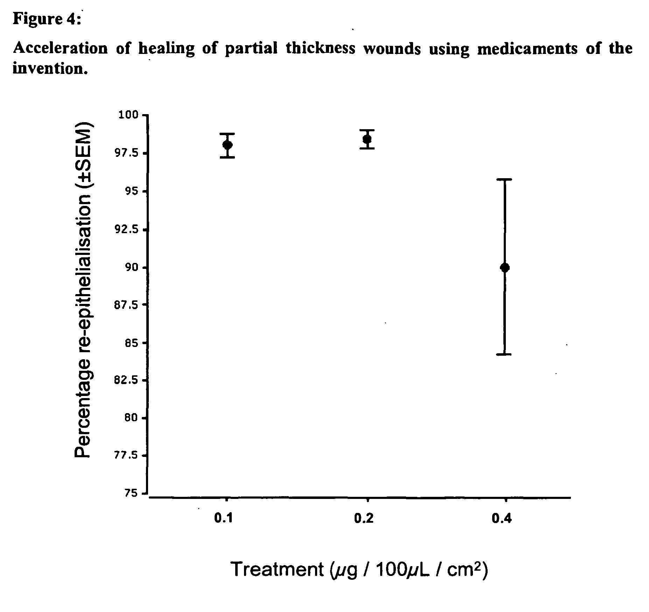

The results of this study are shown in Figure 4.

As can be seen from Figure 4, re-epithelialisation of partial thickness wounds was observed in respect of all concentrations investigated. The greatest extent of re-epithelialisation was observed in wounds treated with medicaments of the invention incorporating 17β-oestradiol at concentrations of 0.1µg 17β-oestradiol/100µl of medicament and 0.2µg/100µl of medicament. Wounds treated with these medicaments exhibited 98.15% re-epithelialisation and 98.56% re-epithelialisation respectively.

The medicaments of the invention found to most effectively promote re-epithelialisation, and hence wound healing, contain concentrations of 17β-oestradiol (constituting the required oestrogen) at concentrations that are five or ten times lower than the minimum effective concentrations suggested by the prior art. Furthermore, the results obtained in this study are consistent with the results of study 3, above, and further indicate that these low concentrations are more therapeutically effective than the amounts suggested in the prior art.

### 5 Medicaments of the invention accelerate healing of full thickness cutaneous excisional wounds in humans

The inventors studied the effects of medicaments of the invention in promoting an increase in the rate of re-epithelialisation (and hence healing) of human punch biopsy wounds.

### 5.1 Materials and methods

### 5.1.1 Medicaments of the invention

Medicaments of the invention were prepared using the constituents described above. Four concentrations of the medicaments were prepared, as follows:
i) 0.02µg 17β-oestradiol/100µl of medicament (0.00002% w/v)
ii) 0.1µg 17β-oestradiol/100µl of medicament (0.0001 % w/v)
iii) 0.2µg 17β-oestradiol/100µl of medicament (0.0002% w/v)
iv) 0.4µg 17β-oestradiol/100µl of medicament (0.0004% w/v)

### 5.1.2 Wound healing model

Healthy male and post-menopausal female volunteers were chosen as the subjects of the study. 44 healthy subjects were recruited to the study, of whom, 40 were healthy males (aged 18-75 years) and 4 were post-menopausal females (aged 53-69 years). The average age of subjects recruited to this study was 42 years. The post-menopausal status of female subjects was confirmed by quantification of serum levels of oestradiol and follicle stimulating hormone (FSH). Serum levels of oestradiol were required to be <90pmol/L, and follicle stimulating hormone levels >31 IU/L.

Each subject had four wounds treated with active drug, one corresponding to each of four concentrations of the medicaments produced. The test medicaments were each administered intradermally as a single dose at a volume of 100µl (sufficient to treat 1cm² of skin) per wound site (the upper, inner aspect of each arm), 10-30 minutes before wounding.

Subjects received 3mm punch biopsies at the sites where the medicaments had been administered. After wounding, all sites received moist wound healing dressings (Standard Care).

### 5.1.3 Assessment of acceleration of wound healing

Biopsy sites were excised, using a 5mm punch biopsy, three days after wounding. Excised wounds were preserved for histological assessment of re-epithelialisation by image analysis. All histological assessments of early wound healing events were made using preserved 5 micron-thickness wound sections taken from the widest part of each excised biopsy site. Sections were stained with Haematoxylin and Eosin to aid visualisation of structural features, and measurements made using image analysis software. The distance travelled by the epithelium is derived from two histological measurements: the freehand total wound diameter and the freehand non-epithelialised wound diameter. The non-epithelialised diameter is subtracted from the total wound diameter, to determine the distance which the new epithelium has covered since the time of wounding. Percentage re-epithelialisation for the new epithelium was calculated as a proportion of the total wound diameter. The average percentage re-epithelialisation value was calculated from the 44 wounds of each treatment group.

### 5.2 Results

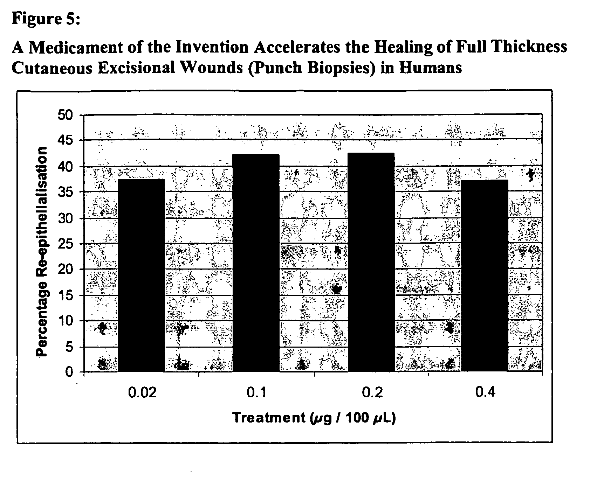

The results shown in Figure 5 illustrate that effective re-epithelialisation of human cutaneous wounds was achieved using all of the medicaments of the invention tested.

The results of this study indicate that preferred acceleration of re-epithelialisation (and hence of wound healing) may be achieved by the administration of between 0.02µg and 0.3µg of 17β-oestradiol to treat an area of approximately 1cm². The medicaments of the invention comprising 0.1µg 17β-oestradiol/100µl of medicament (0.0001% w/v) and 0.2µg 17β-oestradiol/100µl of medicament (0.0002% w/v) demonstrated particularly preferable ability to induce acceleration of re-epithelialisation, and hence wound healing.

## Claims

1. The use of an oestrogen in the manufacture of a medicament, wherein the medicament comprises said oestrogen provided in an injectable carrier solution, the injectable carrier solution comprising an alcohol and a phosphate buffer.

2. The use of an oestrogen in the manufacture of a medicament for accelerating the healing of wounds, wherein the medicament comprises said oestrogen provided in a solution comprising an alcohol and a phosphate buffer.

3. The use according to claim 1 or claim 2, wherein the medicament is for provision to a wound to accelerate wound healing.

4. The use according to any of claims 1 to 3, wherein the oestrogen is selected from the group consisting of:
ethinylyoestradiol, dienoestrol, mestranol, oestradiol, 17β-oestradiol, oestriol, conjugated oestrogens, and phytoestrogens;

5. The use according to any of claims 3 to 4, wherein the medicament provides between 0.005µg and 4µg of the oestrogen per wound centimetre to which the medicament is provided.

6. The use according to claim 5, wherein the medicament provides between 0.02µg and 0.3µg weight of 17β-oestradiol per wound centimetre to which the medicament is provided.

7. The use according to any preceding claim, wherein the alcohol is selected from the group consisting of ethanol or higher water-miscible alcohols such as isopropyl alcohol or benzyl alcohol or other suitable alcohols.

8. The use according to any preceding claim, wherein the solution further comprises a source of chloride ions.

9. The use according to any preceding claim, wherein the solution further comprises a source of potassium ions.

10. The use according to any preceding claim, wherein the pH of the medicament is between 6.7 and 7.7.

11. The use according to any preceding claim, wherein the medicament is for the healing of corneal wounds.

12. The use according to any one of claims 1 to 10, wherein the medicament is for the healing of skin wounds.

13. The use according to any preceding claim, wherein the medicament is for topical administration.

14. The use according to any preceding claim, wherein the medicament is for injection.

15. The use according to claim 14, wherein the medicament is for intradermal injection.

16. The use according to any of claims 3 to 15, wherein the medicament is administered prior to wound formation.

17. The use according to any of claims 3 to 16, wherein the medicament is administered after wound formation.

18. The use according to any of claims 3 to 17, wherein the medicament gives rise to a rate of healing that is at least 10% faster than healing of an untreated wound.

19. The use according to any preceding claim, wherein the medicament is for provision at a graft donor site.

## Patentansprüche

1. Verwendung eines Östrogens bei der Herstellung eines Medikaments, wobei das Medikament das in einer injizierbaren Trägerlösung bereitgestellte Östrogen aufweist, wobei die injizierbare Trägerlösung einen Alkohol und einen Phosphatpuffer aufweist.

2. Verwendung eines Östrogens bei der Herstellung eines Medikaments zur Beschleunigung der Wundheilung, wobei das Medikament das in einer Lösung mit einem Alkohol und einem Phosphatpuffer bereitgestellte Östrogen aufweist.

3. Verwendung nach Anspruch 1 oder Anspruch 2, wobei das Medikament zur Versorgung einer Wunde dient, um die Wundheilung zu beschleunigen.

4. Verwendung nach einem der Ansprüche 1 bis 3, wobei das Östrogen aus der Gruppe ausgewählt ist, die aus Ethinylöstradiol, Dienöstrol, Mestranol, Östradiol, 17β-Östradiol, Östriol, konjugierten Östrogenen und Phytoöstrogenen besteht.

5. Verwendung nach einem der Ansprüche 3 bis 4, wobei das Medikament zwischen 0,005 µg und 4 µg Östrogen pro Zentimeter der Wunde liefert, für die das Medikament bereitgestellt wird.

6. Verwerdung nach Anspruch 5, wobei das Medikament zwischen 0,02 µg und 0,3 µg 17β-Östradiol pro Zentimeter der Wunde liefert, für die das Medikament bereitgestellt wird.

7. Verwendung nach einem der vorstehenden Ansprüche, wobei der Alkohol aus der Gruppe ausgewählt ist, die aus Ethanol oder höheren, mit Wasser mischbaren Alkoholen besteht, wie z. B. Isopropylalkohol oder Benzylalkohol oder anderen geeigneten Alkoholen.

8. Verwendung nach einem der vorstehenden Ansprüche, wobei die Lösung ferner eine Chloridionenquelle aufweist.

9. Verwendung nach einem der vorstehenden Ansprüche, wobei die Lösung ferner eine Kaliumionenquelle aufweist.

10. Verwendung nach einem der vorstehenden Ansprüche, wobei der pH-Wert des Medikaments zwischen 6,7 und 7,7 liegt.

11. Verwendung nach einem der vorstehenden Ansprüche, wobei das Medikament zur Heilung von Homhautwunden dient.

12. Verwendung nach einem der Ansprüche 1 bis 10, wobei das Medikament zur Heilung von Hautwunden dient.

13. Verwendung nach einem der vorstehenden Ansprüche, wobei das Medikament Für topische Applikation vorgesehen ist.

14. verwendung nach einem der vorstehenden Ansprüche, wobei das Medikament zur Injektion vorgesehen ist.

15. Verwendung nach Anspruch 14, wobei das Medikament zur intradermalen Injektion vorgesehen ist.

16. Verwendung nach einem der Ansprüche 3 bis 15, wobei das Medikament vor der Wundbildung verabreicht wird.

17. Verwendung nach einem der Ansprüche 3 bis 16, wobei das Medikament nach der Wundbildung verabreicht wird.

18. Verwendung nach einem der Ansprüche 3 bis 17, wobei das Medikament eine um mindestens 10% höhere Heilungsgeschwindigkeit verursacht als bei der Heilung einer unbehandelten Wunde.

19. Verwendung nach einem der vorstehenden Ansprüche, wobei das Medikament zur Bereitstellung an einer Transplantatentnahmestelle vorgesehen ist.

## Revendications

1. Utilisation d'un oestrogène dans la fabrication d'un médicament, dans laquelle le médicament comprend ledit oestrogène apporté dans une solution de support injectable, la solution de support injectable comprenant un alcool et un tampon de phosphate.

2. Utilisation d'un oestrogène dans la fabrication d'un médicament pour accélérer la cicatrisation de plaies, dans laquelle le médicament comprend ledit oestrogène apporté dans une solution comprenant un alcool et un tampon de phosphate.

3. Utilisation selon la revendication 1 ou la revendication 2, dans laquelle le médicament est destiné à être fourni à une plaie pour accélérer la cicatrisation de la plaie.

4. Utilisation selon l'une quelconque des revendications 1 à 3, dans laquelle l'oestrogène est choisi dans le groupe constitué: d'éthinyloestradiol, de diénoestrol, de mestranol, d'oestradiol, de 17β,oestradiol, d'oestriol, d'oestrogènes conjugués et de phytoestrogènes.

5. Utilisation selon l'une quelconque des revendications 3 à 4, dans laquelle le médicament procure entre 0,005 µg et 4 µg de l'oestrogène par centimètre de plaie pour laquelle le médicament est fourni.

6. Utilisation selon la revendication 5, dans laquelle le médicament procure un poids d'entre 0,02 µg et 0,3 µg de 17β-oestradiol par centimètre de plaie pour laquelle le médicament est fourni.

7. Utilisation selon l'une quelconque des revendications précédentes, dans laquelle l'alcool est choisi dans le groupe constitué d'éthanol ou d'alcools supérieurs miscibles avec l'eau tels que l'alcool d'isopropyle ou l'alcool de benzyle ou d'autres alcools appropriés.

8. Utilisation selon l'une quelconque des revendications précédentes, dans laquelle la solution comprend en outre une source d'ions chlorure.

9. Utilisation selon l'une quelconque des revendications précédentes, dans laquelle la solution comprend en outre une source d'ions potassium.

10. Utilisation selon l'une quelconque des revendications précédentes, dans laquelle le pH du médicament est entre 6,7 et 7,7.

11. Utilisation selon l'une quelconque des revendications précédentes, dans laquelle le médicament est destiné à la cicatrisation de plaies de la cornée.

12. Utilisation selon l'une quelconque des revendications 1 à 10, dans laquelle le médicament est destiné à la cicatrisation de plaies de la peau.

13. Utilisation selon l'une quelconque des revendications précédentes, dans laquelle le médicament est destiné à une administration topique.

14. Utilisation, selon l'une quelconque des revendications précédentes, dans laquelle le médicament est destiné à une injection.

15. Utilisation selon la revendication 14, dans laquelle le médicament est destiné à une injection intradermique.

16. Utilisation selon l'une quelconque des revendications 3 à 15, dans laquelle le médicament est administré avant la formation d'une plaie.

17. Utilisation selon l'une quelconque des revendications 3 à 16, dans laquelle le médicament est administré après la formation d'une plaie.

18. Utilisation selon l'une quelconque des revendications 3 à 17, dans laquelle le médicament donne lien à une vitesse de cicatrisation qui est au moins 10% plus grande que la cicatrisation d'une plaie non traitée.

19. Utilisation selon l'une quelconque des revendications précédentes, dans laquelle le médicament est destiné à être fourni à un site donneur de greffe.
